# EUROPÄISCHE PATENTSCHRIFT

(11) **EP 0 521 925 B1**
(45) Veröffentlichungstag und Bekanntmachung des Hinweises auf die Patenterteilung: **04.05.1994**
(21) Anmeldenummer: 91906253.9
(22) Anmeldetag: 13.03.1991
(51) Int. Cl.: A61K 35/78

(54) **VERWENDUNG VON EFEU ZUR TOPISCHEN BEHANDLUNG DER PSORIASIS**
USE OF IVY FOR TOPICAL TREATMENT OF PSORIASIS
UTILISATION DU LIERRE POUR LE TRAITEMENT TOPIQUE DU PSORIASIS

(30) Priorität: 29.03.1990 DE 4010042
(43) Veröffentlichungstag der Anmeldung: 13.01.1993
(73) Patentinhaber: CASSELLA Aktiengesellschaft, D-60386 Frankfurt (DE)
(72) Erfinder: GREVE, Rainer, D-2360 Bad Segeberg (DE); GREVE, Harald, D-5064 Rösrath 3 (DE); PFADT, Joachim, D-3261 Todesfelde (DE); HÖHLER, Thomas, D-6242 Kronberg 1 (DE)
(74) Vertreter: Muley, Ralf, Dr.
(86) Internationale Anmeldenummer: EP9100470
(87) Internationale Veröffentlichungsnummer: WO9114440

(56) Entgegenhaltungen:
- EP-A- 0 133 151
- FR-A- 2 233 071
- FR-M- 4 324
- GB-A- 2 051 575
- Gabriel Garnier, "Ressources médicinales de la Flore française, Band II, 1961, Vigot Frères, Paris, Seiten 940-943

## Beschreibung

Die vorliegende Erfindung betrifft die Verwendung von Efeu als alleinige Wirksubstanz zur Herstellung eines Arzneimittels zur topischen Behandlung der Psoriasis (Schuppenflechte).

Psoriasis (Schuppenflechte) ist eine äußerst unangenehme Hauterkrankung, von der etwa 1 bis 2 % der Bevölkerung betroffen sind. Es handelt sich offensichtlich um eine epidermale Stoffwechselstörung mit überstürzter Epidermisbildung, wobei die neugebildeten Hautzellen in nur 4 Tagen von der Basalschicht in die Hornschicht wandern, was bei der gesunden Haut 28 Tage dauert. (Pschyrembel, Klinisches Wörterbuch 255. Aufl. 1986, Seite 1380).

Zur Bekämpfung der Psoriasis sind bereits mehrere topische Behandlungsmethoden bekannt, die aber alle mit zum Teil schwerwiegenden Nachteilen behaftet sind.

So ist beispielsweise die früher verbreitete topische Anwendung von Teer-Präparaten, z.B. in Form von Salben und Lotionen, heute wegen unbefriedigender Wirksamkeit und insbesondere wegen möglicher Kanzerogenität obsolet.

Als topischer Standard in der Psoriasis-Therapie gilt heute noch das Dithranol. Die Nebenwirkungen dieser Substanz sind allerdings beträchtlich. Es besteht insbesondere die Gefahr starker Hautreizungen, die sogar den Abbruch der Therapie erforderlich machen können, um die Gefahr einer Hautzerstörung abzuwenden. Im übrigen ist Dithranol in akuten Stadien der Psoriasis, vor allem mit Übergang zu einer Erythrodermie oder einer generalisierten Psoriasis pustulosa kontraindiziert. Die topische Applikation von Retinoiden (beispielsweise Vitamin A-Säure), verursacht bei etwa 75 % der Patienten schwere Nebenwirkungen in Form einer initialen, entzündlichen Verschlechterung des Hautzustandes, der nur mit einer allerdings für die Daueranwendung nicht akzeptablen Kombination mit einem Antibiotikum, wie beispielsweise Erythromycin, entgegengewirkt werden kann (Pharmazeutische Zeitung Nr. 5 vom 01.02.1990, Seite 25).

In neuerer Zeit sind Erfolge mit dem Immunsuppressivum Cyclosporin A erzielt worden. Nachteilig ist hier aber, daß aufgrund der schwerwiegenden Nephrotoxizität der Verbindung eine ständige Kontrolle der Laborparameter des Patienten erforderlich ist (Pharmazeutische Zeitung Nr. 5 vom 01.02.1990, Seite 25). Eine ambulante Therapie ohne ständige ärztliche Überwachung ist somit praktisch ausgeschlossen.

Breit anwendbare, dauerhaft wirksame und nebenwirkungsarme Therapeutika zur Behandlung der Psoriasis stehen bis heute nicht zur Verfügung, die Krankheit gilt als nicht heilbar. Die therapeutischen Maßnahmen beschränken sich bei der Mehrzahl der fast ausschließlich ambulanten Patienten auf eine konservierende, pflegende Behandlung der befallenen Hautareale mit fettenden Cremes oder öligen Zubereitungen, um den oft starken Juckreiz zu lindern und die Austrocknung der Haut zu vermeiden.

Efeu (Hedera helix L.) ist eine in Europa heimische, immergrüne Pflanze, die als Arzneipflanze seit langem bekannt ist. Aufgrund seiner expektorierenden und spasmolytischen Wirkungen wird es insbesondere innerlich gegen Keuchhusten, Asthma und Bronchialkatarrh angewendet (Ebel, Roth, Lexikon der Pharmazie, Thieme Verlag, Stuttgart, 1987). Aber auch als Bestandteil einer Zubereitung zur Behandlung von Zellulitis ist Efeu bereits bekannt (FR-A 2 571 616).

Darüberhinaus beschreibt die EP-A 133 151 eine auch zur Behandlung von Psoriasis geeignete Salbe, die neben einer Vielzahl größtenteils pflanzlicher Bestandteile auch Efeu enthält.

Aufgabe vorliegender Erfindung ist es, ein breit anwendbares Mittel zur nebenwirkungsarmen und dauerhaft wirksamen Behandlung der Psoriasis bereitzustellen.

Diese Aufgabe wird in überraschender Weise gelöst durch die Verwendung von Efeu als alleinige Wirksubstanz zur Herstellung eines Arzneimittels zur topischen Behandlung der Psoriasis (Schuppenflechte).

Zur erfindungsgemäßen Verwendung können alle Teile der Pflanze herangezogen werden, also Wurzeln, Sprosse, Blätter, Blüten und Früchte. Bevorzugt werden allerdings Blätter verwendet. Efeublätter sind als Arzneidroge handelsüblich und somit leicht beschaffbar.

Die Pflanze bzw. die Pflanzenteile können zur erfindungsgemäßen Verwendung als solche eingesetzt werden. Bevorzugt ist allerdings die Verwendung in Form von Extrakten, besonders bevorzugt in Form von medizinischen Zubereitungen, die Efeu oder Efeuextrakte enthalten.

Efeuextrakte können fest oder flüssig sein. Flüssige Extrakte enthalten neben den Wirkstoffen flüssige Auszugsmittel, die dem Fachmann an sich bekannt sind. Bevorzugt sind Wasser, Alkohole, insbesondere Ethylalkohol oder Isopropylalkohol, oder Alkohol-Wasser-Gemische. Feste Extrakte enthalten neben den Wirkstoffen insbesondere inerte Feststoffe, beispielsweise Dextrin oder Siliciumdioxid.

Die Extrakte weisen ein bevorzugtes Droge-Extrakt-Verhältnis von 1 : 2 bis 1 : 0,2 auf.

Die Herstellung der Efeuextrakte erfolgt in dem Fachmann an sich bekannter Weise so, daß mit Hilfe geeigneter, flüssiger Auszugsmittel, insbesondere Wasser, Alkohole oder Alkohol-Wasser-Gemischen, aus einem Teil getrockneter oder frischer Pflanzenmasse 0,1 bis 100 Teile, vorzugsweise 0,2 bis 10 Teile, ganz besonders bevorzugt 1 Teil flüssiger oder 0,2 Teile trockener Extrakt gewonnen werden. Dabei kann die zur Erzielung des jeweils gewünschten Droge-Extrakt-Verhältnisses erforderliche Extraktmasse bei flüssigen Extrakten durch Direktextraktion mit oder ohne nachträgliches Einengen, bei Trockenextrakten durch Eindampfen flüssiger Auszüge zur Trockene und anschließendes Vermischen mit inerten Feststoffen eingestellt werden. Ein bevorzugtes Extraktionsverfahren ist die Perkolation. (DAB 9, Seite 800 in der Monographie "Extrakte").

Medizinische Zubereitungen, die Efeu oder Efeuextrakte enthalten, können flüssig, gelartig oder pasten- bzw. salbenförmig sein und neben dem wirksamen Bestandteil die in der Galenik üblichen, pharmakologisch unbedenklichen und mit den Efeubestandteilen verträglichen Träger- bzw. Verdünnungsmittel und andere Hilfsstoffe enthalten.

Der Anteil an wirksamem Bestandteil, der bevorzugt in Form der flüssigen oder festen Extrakte eingesetzt wird, beträgt, berechnet auf die Masse des pflanzlichen Ausgangsmaterials, 1 bis 100 Prozent, bevorzugt 10 bis 50 Prozent, besonders bevorzugt 25 Prozent.

Hilfs- und Trägerstoffe sind beispielsweise Wasser, pH-puffernde Zusätze wie z.B. Ammoniaklösung und/oder Zitronensäure, niedere Alkohole wie Ethylalkohol, Propylalkohol oder Isopropylalkohole, Diole wie Propylenglycol oder Butandiol, mehrwertige Alkohole wie Glycerol, Paraffinkohlenwasserstoffe, Vaseline, mittelkettige Triglyceride, Ester ein- oder mehrwertiger Alkohole mit höheren pflanzlichen Fettsäuren, polyoxethylierte, mit höheren Fettsäuren teilveresterte mehrwertige Alkohole, unvernetzte und mehr oder weniger stark vernetzte Copolymerisate, insbesondere auf Basis von Acrylsäure, Acrylsäurederivaten oder Vinylpyrrolidon.

Bevorzugt sind Wasser, Isopropylalkohol, Propylenglykol, Glycerol und Polyacrylsäure für Gelzubereitungen und gereinigtes Wollwachs oder Wollwachsalkoholsalbe für Salbenzubereitungen.

Darüberhinaus können die medizinischen Zubereitungen auch noch zusätzliche mikrobiologisch aktive Verbindungen, wie beispielsweise Konservierungsmittel und/oder Antiseptica in pharmazeutisch üblichen Konzentrationen enthalten.

Die Herstellung der medizinischen Zubereitungen erfolgt in an sich bekannter Weise durch Mischen des wirksamen Bestandteils, der vorzugsweise ein flüssiger oder trockener Extrakt aus Efeu ist, mit den gewünschten Träger- und Hilfsstoffen. Bei der Herstellung eines Gel wird z.B. in die vorher fertiggestellte Gelgrundlage ein alkoholisch-wäßriger Fluidextrakt mit ca. 55 Vol% Ethanolgehalt eingerührt und die Mischung homogenisiert.
Zur Herstellung einer Salbe kann ein ebensolcher Fluidextrakt portionsweise in die vorgelegte Menge einer geeigneten Salbengrundlage eingearbeitet und auf einem Salben-Walzenstuhl feinemulgiert werden.

### Beispiel 1

Zur Herstellung eines Fluidextraktes aus Efeublättern im Droge-Extrakt-Verhältnis 1:1 werden 100 g der im Handel erhältlichen getrockneten und geschnittenen Efeublätter nach dem im DAB 9 Seite 800 in der Monographie "Extrakte" beschriebenen Verfahren durch Perkolation extrahiert. Die Droge wird dabei zuerst vermittels einer geeigneten Mühle grob gepulvert, mit 30 g Ethanol 70 Vol% in einer offenen Schale gleichmäßig durchfeuchtet und bedeckt 2 Stunden zum Vorquellen stehen gelassen. Danach wird gesiebt, das Gemisch in den vom DAB 9 vorgeschriebenen Perkolator gefüllt und nach dem dort angegebenen Verfahren die Perkolation mit Ethanol 70 Vol% durchgeführt. Diese ist beendet, wenn 85 g Perkolat abgetropft sind. Dann wird der Ablaufhahn geschlossen, die weitere Aufgabe von Ethanol 70 Vol% unterbrochen und der Drogenrückstand abgepreßt. Die Preßflüssigkeit wird filtriert, am Rotationsverdampfer unter Vakuum zu 15 g eingeengt und zu den 85 g Perkolat gegeben. Nach mehrtägigem Stehen im geschlossenen Gefäß bei etwa 5 bis 10°C ist das Produkt auf Klarheit zu prüfen und ggf. zu filtrieren. Man erhält 100 g Efeublätterfluidextrakt 1:1, eine dunkelgrünbräunliche Flüssigkeit von arteigenem und ethanolischem Geruch. Das Produkt wird in Braunglasflaschen abgefüllt und unverdünnt angewendet oder zu anderen medizinischen Zubereitungen weiterverarbeitet. Anwendung: Alle 12 Stunden die befallenen Hautbezirke mit einem getränkten Wattebausch abtupfen.

### Beispiel 2

Zur Herstellung einer fettfreien Gelzubereitung werden 43 g gereinigtes Wasser in einem 200 ml-Becherglas mit 5 g Propylenglycol gemischt, sodann 1,75 g Polyacrylsäure (z.B. Carbopol 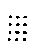 950) auf die Flüssigkeitsoberfläche gestreut und mit einem Glasstab eingerührt. Die Mischung wird bedeckt mindestens 24 Stunden zwecks Gelbildung zum Quellen stehen gelassen. Danach wird durch Zusatz von 0,25 g Ammoniaklösung 10 % der pH-Wert des Quellgemisches auf 4 bis 5 eingestellt und 5 g Glycerol 85% eingearbeitet. Dann fügt man 25 g Efeublätterextrakt 1:1 (nach Beispiel 1) in kleinen Anteilen hinzu, wobei nach jeder Zugabe eine Zeitlang gerührt wird. Zuletzt folgen 20 g Isopropylalkohol, und das Produkt wird homogen gerührt. Man erhält ein dunkelgrünes, klares dickflüssiges Gel mit einem Gehalt von 25 Prozent wirksamem Bestandteil, berechnet auf die Masse des Pflanzlichen Ausgangsmaterials. Das Gel wird in Braunglasflaschen oder Tuben abgefüllt. Anwendung: 2 bis 3 mal täglich auf die betroffenen Hautareale auftragen und bis zum Verschwinden leicht einmassieren.

### Beispiel 3

Zur Herstellung einer Salbe mit zusätzlich fettendem Effekt werden 75 g Wollwachsalkoholsalbe in eine Salbenschale mit Pistill eingewogen und weichgerührt. Dann werden 25 g Efeublätterfluidextrakt 1:1 (nach Beispiel 1) in kleinen Anteilen zu der Salbenmasse gegeben und eingearbeitet. Zuletzt wird durch kräftiges Rühren oder ggf. auf einem Walzenstuhl homogenisiert. Man erhält eine weiche, grüne Salbe mit 25 Prozent wirksamem Bestandteil, berechnet auf die Masse des pflanzlichen Ausgangsmaterials, die in Tuben aus Kunststoff oder Metall abgefüllt wird. Anwendung: 2 bis 3 mal täglich auf die erkrankten Hautpartien auftragen und leicht verreiben.

### Beispiel 4

Zur Herstellung einer fettenden Salbe mit erhöhtem Wirkstoffgehalt werden in einer Salbenschale mit Pistill 10 g gepulverter Efeutrockenextrakt 1 : 0,2 mit 10 g wasserhaltigem Wollwachs (Lanolin) homogen und klümpchenfrei verrieben. Zu dieser Verreibung werden weitere 80 g Lanolin in kleinen Anteilen hinzugemischt und die Masse homogen gerührt. Man erhält 100 g einer bräunlich-gelben weichen Salbe, die in Tuben aus Metall oder Kunststoff abgefüllt wird. Der Gehalt an wirksamem Bestandteil, berechnet auf das pflanzliche Ausgangsmaterial, beträgt 50 Prozent.
Anwendung: Alle 12 Stunden, bei Beginn der Behandlung auch dreimal täglich, dünn auf den erkrankten Hautarealen verstreichen und leicht aber gründlich einreiben.

### Beispiel 5

Zur Herstellung einer flüssigen Zubereitung für den kurzfristigen Verbrauch werden in einem 200 ml-Becherglas mit Rührstab 30 g Ethanol 96 Vol% und 30 g gereinigtes Wasser gemischt. Mit Anteilen dieser Mischung werden in einer Reibschale 8 g Efeutrockenextrakt 1 : 0,2 mittels Pistill angerieben, in das Becherglas übergeführt und durch Rühren gelöst. Die Lösung wird mit Glycerol zu 100 g aufgefüllt und homogenisiert. Man erhält eine grünlichbraune Lösung, die man 24 Stunden an einem kühlen Ort unter Verschluß absetzen läßt, dann filtriert und danach in dem Verbrauch angemessene Braunglasflaschen füllt. Der Gehalt, berechnet auf das pflanzliche Ausgangsmaterial, beträgt 40 Prozent.
Anwendung: 2 bis 3 mal täglich mit einem Wattebausch auf die psoriatischen Hautpartien auftragen und gut verteilen.

### Beispiel 6

Für einen freiwilligen Selbstversuch wurde Efeublätterfluidextrakt 1 : 1, wie im Beispiel 2 beschrieben zu einer 25-prozentigen Gelzubereitung verarbeitet. Behandelt wurde eine seit 17 Jahren bestehende generalisierte Psoriasis mit auffälliger symmetrischer Ausbreitung am Rumpf. Der betroffene Patient ist Mediziner und behandelte streng einseitig links bei nur einmal täglicher Applikation. Die rechte Seite wurde, wie bisher, nur mit indifferentem Babyöl gepflegt. Nach etwa 6 Wochen traten erste Anzeichen einer Besserung der behandelten Hautbezirke gegenüber der unbehandelten Gegenseite ein, Juckreiz und Schuppung nahmen ab. Nach einer sechsmonatigen Behandlung war eine wesentliche Besserung eingetreten und weite vorher befallene Hautbezirke vollkommen abgeheilt. Unerwünschte Nebenwirkungen konnten zu keinem Zeitpunkt beobachtet werden.

## Patentansprüche

1. Verwendung von Efeu als alleinige Wirksubstanz zur Herstellung eines Arzneimittels zur topischen Behandlung der Psoriasis (Schuppenflechte).

2. Verwendung gemäß Anspruch 1, dadurch gekennzeichnet, daß Efeu in Form von Extrakten eingesetzt wird.

3. Verwendung gemäß Anspruch 1, dadurch gekennzeichnet, daß Efeu in Form medizinischer Zubereitungen eingesetzt wird.

4. Verwendung gemäß Anspruch 3, dadurch gekennzeichnet, daß die medizinischen Zubereitungen flüssig, gelartig oder pasten- bzw. salbenförmig sind.

5. Verwendung gemäß Anspruch 3 und/oder 4, dadurch gekennzeichnet, daß die medizinischen Zubereitungen einen Anteil an wirksamem Bestandteil, berechnet auf die Masse des pflanzlichen Ausgangsmaterials, von 1 bis 100 Prozent, bevorzugt 10 bis 50 Prozent, besonders bevorzugt 25 Prozent, aufweisen.

## Claims

1. Use of ivy as sole active substance for preparing a medicament for the topical treatment of psoriasis.

2. Use according to claim 1, characterized in that ivy is employed in the form of extracts.

3. Use according to claim 1, characterized in that ivy is employed in the form of medicinal preparations.

4. Use according to claim 3, characterized in that the medicinal preparations are liquid, gel-like or in the form of a paste or ointment.

5. Use according to claim 3 and/or 4, characterized in that the medicinal preparations contain a quantity of active substance, relative to the mass of the vegetable starting material, of 1 to 100 percent, preferably 10 to 50 percent, particularly preferably 25 percent.

## Revendications

1. Utilisation du lierre en tant que principe actif unique pour préparer un médicament destiné au traitement local du psoriasis.

2. Utilisation selon la revendication 1, caractérisée en ce que le lierre est utilisé sous forme d'extraits.

3. Utilisation selon la revendication 1, caractérisée en ce que le lierre est utilisé sous forme de préparations médicinales.

4. Utilisation selon la revendication 3, caractérisée en ce que les préparations médicinales sont liquides ou se présentent sous forme d'un gel ou d'une pâte ou d'une pommade.

5. Utilisation selon la revendication 3 et/ou 4, caractérisée en ce que les préparations médicinales contiennent une quantité du principe actif, calculée par rapport à la masse de la matière végétale de départ, de 1 à 100 %, de préférence de 10 à 50 % et tout particulièrement de 25 %.
